# EUROPEAN PATENT APPLICATION

(11) **EP 2 873 735 A1**
(43) Date of publication of application: **20.05.2015**
(21) Application number: 14152191.4
(22) Date of filing: 22.01.2014
(51) Int. Cl.: C12N 15/82, A01H 5/00

(54) **Method and materials for making plants heat-resistant**

(30) Priority: 14.11.2013 EP 13192897
(71) Applicant: Universität Potsdam, 14469 Potsdam (DE)
(72) Inventor: Bäurle, Isabel, 14469 Potsdam (DE)
(74) Representative: Hoppe, Georg Johannes

(57) **Abstract**

The invention pertains to a method for generating a heat-resistant plant, comprising the step of introducing a polynucleotide, comprising a first sequence portion for transcriptionally driving a second sequence portion that is operably linked to a second sequence portion encoding for a miRNA156 family member into a plant cell, wherein the first sequence portion is heat-stress inducible.

## Description

The invention pertains to a polynucleotide for making plants more resistant to heat stress as well as to plants containing such a polynucleotide. The polynucleotide comprises a first sequence portion that is transcriptionally driving a second sequence portion, which is encoding a miRNA of the plant miRNA156 family.

### Background

To cope with changes in their physical environment, plants have evolved physiological responses allowing them to tolerate stressful conditions such as extreme temperatures, drought and high salinity (Jenks MA & Hasegawa PM (2005) Plant Abiotic Stress (Blackwell, Oxford)). These abiotic stresses are a major limiting factor for the geographical distribution and productivity of plants. Hence, stress tolerance is a central goal in crop breeding.

In plants, double-stranded RNA (dsRNA) is an effective trigger of RNA silencing, and several classes of endogenous small RNA (sRNA), processed from dsRNA substrates by endonucleases, are essential in controlling gene expression. One such sRNA class is the microRNAs (miRNAs).

MiRNAs are ca. 21 nucleotide long single-stranded RNA molecules that guide effector proteins, most commonly the ARGONAUTE1 (AGO1) protein, to complementary mRNA causing either mRNA cleavage or translational inhibition (Axtell MJ (2013) Classification and Comparison of Small RNAs from Plants. Ann Rev Plant Biol 64:137-159; Voinnet O (2009) Origin, biogenesis, and activity of plant microRNAs. Cell 136(4):669-687). MiRNAs are generated from primary transcripts that are transcribed by RNA polymerase II and fold into a hairpin structure that is cleaved to release the active miRNA (Rogers K & Chen X (2013) Biogenesis, Turnover, and Mode of Action of Plant MicroRNAs. Plant Cell 25:2383-2399). MiRNAs play important roles during plant growth, developmental transitions and determination of cell identity (Voinnet O (2009) Origin, biogenesis, and activity of plant microRNAs. Cell 136(4):669-687; Rogers K & Chen X (2013) Biogenesis, Turnover, and Mode of Action of Plant MicroRNAs. Plant Cell 25: 2383-2399; Jones-Rhoades MW, Bartel DP, & Bartel B (2006) MicroRNAS and their regulatory roles in plants. Ann Rev Plant Biol 57:19-53). MiRNAs are also involved in the modulation of plant responses to biotic and abiotic stresses (Khraiwesh B, Zhu JK, & Zhu J (2012) Role of miRNAs and siRNAs in biotic and abiotic stress responses of plants. Biochim Biophys Acta 1819(2):137-148; Sunkar R, Li YF, & Jagadeeswaran G (2012) Functions of microRNAs in plant stress responses. Trends Plant Sci 17(4):196-203; Kruszka K, et al. (2012) Role of microRNAs and other sRNAs of plants in their changing environments. J Plant Physiol 169(16):1664-1672; Fujii H, Chiou TJ, Lin SI, Aung K, & Zhu JK (2005) A miRNA involved in phosphate- starvation response in Arabidopsis. Curr Biol 15(22):2038-2043; Navarro L, Jay F, Nomura K, He SY, & Voinnet O (2008) Suppression of the microRNA pathway by bacterial effector proteins. Science 321(5891):964-967; Sunkar R & Zhu JK (2004) Novel and stress-regulated microRNAs and other small RNAs from Arabidopsis. Plant Cell 16(8):2001-2019).

One object of the present invention was to find a means of making plants more resistant to heat stress.

### Description of the invention

The inventors discovered a function for miRNAs of the miR156 family in the heat stress (HS) response of plants. Members of the miR156 family are highly induced after HS and are functionally important for the HS memory. Members of the miR156 family promote sustained expression of a set of HS-responsive genes, and are critical only after HS, demonstrating that the effects of modulating miR156 levels on HS memory do not reflect pre-existing developmental alterations.

Accordingly, in a first aspect, the invention refers to a polynucleotide that can be used to generate heat-resistant plants (when compared to wild-type plants).

Such a polynucleotide comprises a first sequence portion that is able to transcriptionally drive a second sequence portion, wherein the fist sequence portion is operably linked to a second sequence portion that encodes for a miRNA of the miRNA156 family. The first sequence portion is in one embodiment of the invention a heat-stress inducible promotor that allows for the transcription of a miRNA of the miRNA156 family, such as miRNA156, only after an initial heat stress. This reduces any possible negative effect that the expression of miRNA15 might have on the growth of the plant.

Since the first sequence portion is heat-stress inducible, it allows for the transcriptional activation of the second sequence portion, i.e. the transcription of a miRNA of the miRNA156 family, only after the plant was subject to heat stress.

The term "heat stress", as used herein, refers to elevated growth temperatures that occur temporarily which are above the growth optimum temperature and cause potential denaturing of proteins and inhibition of physiological processes. A molecular marker of heat stress is the induction of heat shock protein (HSP) gene expression, as known in the art. HSP induction can be measured with any method suitable and known in the art, such as polymerase chain reaction (PCR) or Western blotting. Generally, temperatures above 33 °C are considered as heat stress. Time periods of at least 10 minutes may lead to heat stress.

The term "heat stress inducible promotor", as used herein, refers to the expression of RNA from such a promotor that is induced only under heat stress. At the growth optimum temperature, RNA expression from a heat stress inducible promotor is not or only marginally detectable. Upon heat stress, the promotor becomes active and leads to the transcription of the sequence portion to which it is operably linked.

In one embodiment of the invention, the first sequence portion is a promotor selected from the group consisting of: HSP21, HSA32, HSP17, HSP18, HSP22, and HSP70. As examples, the sequences of the HSP21 and the HSP17 promotor of Arabidopsis thaliana are provided as SEQ ID NO. 8 and 9, respectively. The sequences of the other HSP promoters, also from other plants, are known in the art and can be retrieved, for example, from a suitable database. The coding sequences of heat shock proteins are highly conserved among different plants (Kotak S, Larkindale J, Lee U, von Koskull-Doring P, Vierling E, Scharf KD (2007) Complexity of the heat stress response in plants. Curr Opin Plant Biol 10: 310-316; Siddique M, Gernhard S, von Koskull-Doring P, Vierling E, Scharf KD (2008) The plant sHSP superfamily: five new members in Arabidopsis thaliana with unexpected properties. Cell stress & chaperones 13: 183-197; Scharf KD, Berberich T, Ebersberger I, Nover L (2012) The plant heat stress transcription factor (Hsf) family: structure, function and evolution. Biochimica et biophysica acta 1819: 104-119; Arazi T, Talmor-Neiman M, Stav R, Riese M, Huijser P, Baulcombe DC (2005) Cloning and characterization of micro-RNAs from moss. Plant J 43: 837-848; Axtell MJ, Snyder JA, Bartel DP (2007) Common functions for diverse small RNAs of land plants. Plant Cell 19: 1750-1769; Cho SH, Coruh C, Axtell MJ (2012) miR156 and miR390 regulate tasiRNA accumulation and developmental timing in Physcomitrella patens. Plant Cell 24: 4837-4849; Xin M, Wang Y, Yao Y, Xie C, Peng H, Ni Z, Sun Q (2010) Diverse set of microRNAs are responsive to powdery mildew infection and heat stress in wheat (Triticum aestivum L.). BMC plant biology 10: 123; Yu X, Wang H, Lu Y, de Ruiter M, Cariaso M, Prins M, van Tunen A, He Y (2012) Identification of conserved and novel microRNAs that are responsive to heat stress in Brassica rapa. J Exp Bot 63: 1025-1038).

The promotor may comprise regulatory sequences, such as enhancers and cis-elements.

The present invention can be practiced with all kind of plants. The sequences of miRNAs of the miRNA156 family are well-conserved among plant varieties and species. Therefore, a person of skill in the art can construct a polynucleotide with homologous sequences for the use in a particular plant variety or species based on the information provided herein together with his or her general knowledge in the art.

The term miRNA156 family, as used herein, refers to a polynucleotide in the form of the second sequence portion that is selected from the group consisting of: miRNA156a-f, miRNA156g, miRNA156h, and miR157. Such polynucleotides are present in all plants, since miRNA156 family is well-conserved among different plant varieties and species.

The miRNA156 family also refers to functional fragments of the miRNAs listed above. A functional fragment is a polynucleotide that is shorter than miRNA156a-f, miRNA156g, miRNA156h, and miR157, respectively, but exerts the same physiological function in making a plant heat resistant.

In plants, a single miRNA small RNA (sRNA) silencing signal is processed from a long precursor transcript of nonprotein-coding RNA, termed the primary miRNA (pri-miRNA). A region of the pri-miRNA is partially self-complementary allowing the transcript to fold back onto itself to form a stem-loop structure of (possibly imperfectly) double-stranded RNA (dsRNA).

Accordingly, in one embodiment, the second sequence portion comprises or consists of a natural immature miRNA sequence that allows for the formation a secondary structure, in particular a stem-loop structure, of the transcript.

Artificial miRNA (amiRNA) technology uses endogenous pri-miRNAs, in which the miRNA and miRNA* (a passenger strand of the miRNA duplex) sequences have been replaced with corresponding amiRNA/amiRNA* sequences that direct highly efficient RNA silencing of the targeted gene (Ossowski, S., Schwab, R., and Weigel, D., Gene silencing in plants using artificial microRNAs and other small RNAs. Plant J. 2008; 53(4): 674-690; Eamens, A.L., McHale, M., and Waterhouse, P.M. The Use of Artificial MicroRNA Technology to Control Gene Expression in Arabidopsis thaliana. Methods Mol Biol. 2014; 1062:211-224). Accordingly, in certain embodiments of the invention, the second sequence portion may also be, at least in part, artificially engineered.

Put differently, in one embodiment of the invention, the second sequence portion comprises a sequence that encodes a mature miRNA sequence that does not form a secondary structure by itself. In this case, the second sequence portion comprises sequences that allow for the formation of a stem-loop structure. Such a stem-loop structure is advantageous for the correct processing and localization of the miRNA within the plant cell. The sequences that allow for the formation of a stem-loop structure can be natural or artificial.

Examples of polynucleotides of the invention are combinations of SEQ ID NO. 8 operably linked to a sequence of SEQ ID NO. 1, SEQ ID NO. 2, SEQ ID NO. 3, SEQ ID NO. 4, SEQ ID NO. 5, SEQ ID NO. 6, or SEQ ID NO. 7. Further examples of polynucleotides of the invention are combinations of SEQ ID NO. 9 operably linked to a sequence of SEQ ID NO. 1, SEQ ID NO. 2, SEQ ID NO. 3, SEQ ID NO. 4, SEQ ID NO. 5, SEQ ID NO. 6, or SEQ ID NO. 7.

The polynucleotide is selected preferably from the group consisting of desoxyribo nucleic acid (DNA), and chemically modified, i.e. artificial DNAs such as Peptide Nucleic Acid (PNA) or morpholinos.

The backbone of PNAs is composed of repeating N-(2-aminoethyl)-glycine units linked by peptide bonds. The various purine and pyrimidine bases are linked to the backbone by a methylene bridge (-CH₂-) and a carbonyl group (-(C=O)-) (Nielsen PE, Egholm M, Berg RH, Buchardt O (December 1991). Sequence-selective recognition of DNA by strand displacement with a thymine-substituted polyamide. Science 254 (5037): 1497-500).

Morpholinos have standard nucleic acid bases that are bound to morpholine rings instead of deoxyribose rings and linked through phosphorodiamidate groups instead of phosphates (Summerton, J; Weller D (1997). "Morpholino Antisense Oligomers: Design, Preparation and Properties". Antisense & Nucleic Acid Drug Development 7 (3): 187-95).

In another aspect, the invention refers to a vector (i.e. a small piece of DNA that can be stably maintained in an, and into which a polynucleotide of the invention can be inserted for cloning and expression purposes) comprising a polynucleotide as described above and herein. Such a vector may be a plasmid, a cosmid or an artificial chromosome. The vector can be used to provide a polynucleotide of the invention in a living plant cell, in a plant tissue or a whole plant. The vector may generate a miRNA of the miRNA156 family upon heat-stress in cells of the plant.

The vector may remain episomal in a plant cell, or integrates into the genome of the plant cell.

Expression constructs of the invention may optionally contain a transcription termination sequence and/or enhancer elements. Transcription termination sequences can be positioned downstream of a coding sequence to provide for efficient termination. Classical enhancers are cis-acting elements that increase gene transcription and can also be included in the expression construct. Classical enhancer elements are known in the art, and include, but are not limited to, the CaMV 35S enhancer element, cytomegalovirus (CMV) early promoter enhancer element, and the SV40 enhancer element.

The vector of the invention can be replicated and isolated using any method known in the art.

In another aspect, the invention refers to a heat-resistant plant cell (i.e. a single cell, isolated or as part of a group of cells, comprising plant tissues or whole plants), which comprises a polynucleotide as described above and herein.

Such a heat resistant plant cell comprises at least one polynucleotide as described above and herein, comprising a heat-inducible promotor operably linked to a sequence of the miRNA156 family.

In another aspect, the invention refers to a method for generating a heat-resistant plant. Such a method comprises the step of introducing at least one polynucleotide as described above and herein, with a HS-inducible promotor upstream of a sequence of the miRNA156 family and operably linked thereto, into a plant cell.

The introduction into the plant cell can be performed using any suitable method known in the art. Techniques for transforming plant cells with a gene include, for example, Agrobacterium infection, biolistic methods, electroporation, calcium chloride treatment, PEG-mediated transformation, etc. Transformed cells can be selected, redifferentiated, and grown into plants that contain and express a polynucleotide of the invention using standard methods known in the art. The seeds and other plant tissue and progeny of any transformed or transgenic plant cells or plants of the invention are also included within the scope of the present invention.

The subject invention also concerns methods for producing a plant that exhibits increased heat stability relative to a wild type plant, wherein a polynucleotide of the invention, encoding a miRNA of the miRNA156 familiy under the control of a heat-inducible promotor is introduced into a plant cell and the polypeptide(s) encoded by the polynucleotide(s) is expressed.

In yet another aspect, the invention refers to a method of increasing resistance of a plant to heat stress conditions comprising the step of introducing a polynucleotide as described above and herein into a plant cell.

In an alternative embodiment, it is also possible to generate a polynucleotide as described above and herein in a plant cell. For example, a sequence of the miRNA156 family in the form of a stem-loop-precursor can be introduced at the 3' side of an endogenous promotor, such that promotor and miRNA are operably linked and the miRNA can be expressed from the endogenous heat-inducible promotor. Alernatively, it is possible to introduce a heat-inducible promotor at the 5' side of an endogenous miRNA member of the miRNA156 family such that the endogenous miRNA is under the transcriptional control of the introduced promotor.

The replacement of endogenous sequences can be achieved using methods known in the art, which are based on homologous recombination.

In another aspect, the invention pertains to a method for generating a heat-resistant plant, as described herein, comprising the step of generating a polynucleotide as described herein in a plant cell or of introducing a polynucleotide as described herein into a plant cell.

In a further aspect, the invention pertains to a kit for generating a heat-resistant plant. Such a kit may comprise a vector comprising a polynucleotide of the invention, optionally together with other components, e.g., oligonucleotide probes and primers, nucleotides, buffer solutions, etc.

The oligonucleotide probes and primers can be polymerase chain reaction (PCR) primers that can hybridize to the polynucleotide of the invention. Oligonucleotide probes of the invention can be used in methods for detecting and quantitating nucleic acid sequences encoding a miRNA of the miRNA156 family. Oligonucleotide primers of the invention can be used in PCR methods and other methods involving nucleic acid amplification. Probes and primers of the invention can optionally comprise a detectable label or reporter molecule, such as fluorescent molecules, enzymes, radioactive moiety (e.g., 3H, 35S, 1251, etc.), and the like. Probes and primers of the invention can be of any suitable length for the method or assay in which they are being employed. Typically, probes and primers of the invention will be 10 to 500 or more nucleotides in length.

The invention also refers to the use of such a kit for generating a heat-resistant plant.

### Sequences

**SEQ ID NO. 1:** miRNA156a-f
   ath-miR156a
   TGACAGAAGAGAGTGAGCAC
**SEQ ID NO. 2:** miRNA156g
   ath-miR156g
   CGACAGAAGAGAGTGAGCAC
**SEQ ID NO. 3:** miRNA156h
   ath-miR156h
   TGACAGAAGAAAGAGAGCAC
**SEQ ID NO. 4:** miRNA156a
   ath-miR157a
   TTGACAGAAGATAGAGAGCAC
**SEQ ID NO. 5:** miRNA156b
   ath-miR157b
   TTGACAGAAGATAGAGAGCAC
**SEQ ID NO. 6:** miRNA156c
   ath-miR157c
   TTGACAGAAGATAGAGAGCAC
**SEQ ID NO. 7:** miRNA156d
   ath-miR157d
   TGACAGAAGATAGAGAGCAC
**SEQ ID NO. 8:** HSP21
   AthHSP21 promotor sequence 
TCAAGAAATTAGGGTTCCCAGATGAAAAAGAGGATATGTTAGCAGAAGACGGAAACAAATTTGGTTGTTCTTGTGGA
CGACGACAGAGCTTTTGCCTTGGTGGGGGTTTCTCGAATACTTGGAGATCGAGGAAAGGAGAAGCTGAAATTACCGA
AATAGCCTTAGCTGCACCGTCGTAAGTCGTGTAACTAGATTGTGTCAAGAAGGGCCTTTAATAGGCCTTCAATTCAGA
ATGTTCTAAAACCGAATTAAAGTACTGTTCGGCGTCCACTAGATTTTGTAGGTACTATTGCATCATCATCATCTTATATC
AGTTCAATGTGTGTCTTGTTTTTTCTTTTTTAGTCTATTACATCATCTGATCTTATTCTAGCAGCCTTTTGAAAATATAGA
GAAAAATCATTCAATGTTTCTAAAACCAAATCTATACACAAAACATAAAGTCTTTTGGATCATTGAACATTTGATCGGA
TTGCGGATTAATAGATTAGAGCTTAAATTTTGTATAATTTTATACCAATCAAAATTAATATAGAGTCATGTCATGCTATT
TGTATAATTTATGTACAACAATTATTATATAAAACGACTTGAACAATTCTGTGATAAAGAAAAAAAAAAAAAAAAACGAC
 
TTGAACAATTCAACATGCTATAGATAGTTTATACGTAAGCTAATAAGCCTGTTTGATACTGTATATGACATACGTGTAG
CTGATATGATCAAACTAGACAAAATTTTCTAATTTCTCCCGTAGATATATATTTTTAGTTTACTTGGGCATTCCATTTCTC
ATATGTTTCTGCAAGTTAGCACACATAATGGTGACTTGGTGAGTTGCTTTTTTCTCTCTCTTTTTCAATATGATATGCTT
TCTTCGATTTTTTTATTTTTTTTAAACAATATATCATCTAATCATGGTTTTTTCGTAATCGTGGTTTTTCTAATTGTAGAAA
TCTAGTCTTTTTTTTTCGTTTATTTTGTTTTTATTAGTTCATCACAAATAAACACTGATTGGCAAGAAGGAAAAAAAGAA
GATGGTTCGGGAGAAGATGAGAAAACACGAGAAAACGCACAAAGGTTAACAAATAGTAAAATAGATAGATAGAAAA
CAAAACGAGTCGAGAAACTTCGTATCAAATCTTCAACCTTCAAGACCTCTCCTTTTGTCACTTAGAGCCTTCTCATCCTC
ACCTACATATATACACTCACTTCTACAATTGTAGTAGATACTCAACAAACTCAGAACTAGAATTTTCTAATCTTCACTCA
GCGATATTTTTGGCTCATACTCGAAACAA
**SEQ ID NO. 9:** HSP17
   Hvu HSP17 promotor sequence 
GTAGAGAATTTTAAGATGTCCATGACCACCCGACCACCCGCTAGCTGCGTCATTGTGTAGTACTCTTTCTGTTTTTAAAA
TAAGTGTCTGAACTTTATACTAACTTTAATATAAAATTGTACTAAACTTGAGACATTTATTTAGACGGAGGGAGTAGTA
TTTATTTACGTTTAACTATTGTACTTGCTCGCTGTAAACTTTAATGACAGATAACCTCTACTGTTAATCATGCTATTCTGG
AAACGGCCCCACAACCCATTCTTGAATCTAGACTAGTCGCCCCCTTGCTGGAATTATACAATTATTTATAGAAAAATCTC
ACAAAAAAATTATTAACAAAAAAAAAAGGGAGAAGGCGCCCTAACGGCAGGTTCGTGAGTCCCCTGGAAGACGATA
GACCCTTCGAGGGTTGGGAACAGGAAAGAACGAATGGCTCACAGCCTACCTGGAAAGCGAAAGAGAAACAAACACT
CGTGGATTGGACAGTCCTCGAGATTCTTCAAGATCAGTACCTACGATGCTATAAGTAGTGGCTCACCTTCCATCGCCCT
CCCAACTCAAGCCAGCCAGCGTCAAACCAAAGCGAACAACATCCTACCTACAATCCGATACAGATTAATTTCAACGCG
AGCTACAAACCAAAGCAAGCAACACCGACA
**SEQ ID NO. 10:** Stem loop sequence Barley (Hordeum vulgare)
   hvu-MIR156 
UGACAGAAGAGAGUGAGCACACAGCGGCAGGCUACGUCUGAUAAGAGCAUGGUUAAUAGUAUAGCCAACUGC
UGGCUAUACUAUGAGCACGUGCUUAGACGCAGCUGCUGUUGCGUGCUCACCCUCUCUCUGUCU
**SEQ ID NO. 11:** mature miRNA Barley (Hordeum vulgare)
   hvu-miR156
   UGACAGAAGAGAGUGAGCACA
**SEQ ID NO. 12:** mature miRNA Brassica napus
   bna-miR156a
   UGACAGAAGAGAGUGAGCACA

### Examples

### Materials and Methods

### Plant material and growth conditions

Plants were grown in GM medium (1 % (w/v) glucose) at 23 °C/ 21 °C 16 h light/ 8h dark cycles unless otherwise indicated. *ago1-25, ago1-27,* and *dcl1-9* were obtained from N. Baumberger and C. Dean, respectively. *SPL9::SPL9-GUS, SPL9::rSPL9-GUS* and *suo-2* (Yang L, Wu G, & Poethig RS (2012) Mutations in the GW-repeat protein SUO reveal a developmental function for microRNA-mediated translational repression in Arabidopsis. Proc Natl Acad Sci U S A 109(1):315-320) were obtained from S. Poethig. *35S::MIM156* and *35S::MIMIPS1 Agrobacterium* stocks, *hsp101* (N566394), *hsfa2* (N508978) were obtained from the Nottingham Arabidopsis Stock Centre. Heat stress treatments were performed in a waterbath or incubator. For the priming HS (ACC), 3-4d old seedlings were subjected to a heat regime of 1 h 37 °C, 1.5 h 23 °C, 45 min 44 °C, except where a short ACC was applied omitting the 44 °C step. A tester HS was applied 2-3 d later for 45-110 min. For acquisition of thermotolerance the regime was 1 h 37 °C, 1.5 h 23 °C, 0-180 min 44 °C; basal thermotolerance was assayed by incubation for 0-60 min at 44 °C. After heat treatment, plants were returned to normal growth conditions until analysis. For analysis of gene expression during maintenance of acquired thermotolerance, 3-4 d old seedlings were subjected to ACC or mock treatment and harvested at the indicated time points.

### Construction of transgenic lines

For *SPL2::rSPL2,* a 3.9kb promoter fragment was amplified with primers (all primer sequences are given in Table 1 and in the sequence listing) introducing *Nco*I and *Sac*I restriction sites. The coding sequence and 3' region (2.5 kb) was amplified mutating the miRNA binding site while preserving the amino acid sequence with primers introducing *Sac*I and *Xma*I sites (Wang JW, Schwab R, Czech B, Mica E, & Weigel D (2008) Dual effects of miR156- targeted SPL genes and CYP78A5/KLUH on plastochron length and organ size in Arabidopsis thaliana. Plant Cell 20(5):1231-1243). After sequencing, the *SPL2::rSPL2* construct was assembled in *pUC-ML939* and transferred via *Asc*I into *pBarMAP-ML516.* For *SPL11::rSPL11,* a 2.6 kb promoter fragment was amplified with primers introducing Ncol and *Sac*I restriction sites. The coding sequence and 3' region (2.5 kb) was amplified mutating the miRNA binding site while preserving the amino acid sequence with primers introducing *Sac*I and *Xma*I sites. After sequencing, the *SPL11::rSPL11* construct was assembled in *pUC-ML939* and transferred via *Asc*I into *pBarMAP-ML516.* Transgenic plants were generated using the floral dip method (Clough SJ & Bent AF (1998) Floral dip: a simplified method for Agrobacterium-mediated transformation of Arabidopsis thaliana. Plant J 16(6):735-743). For *35S::MIR156h,* a 0.5 kb fragment encompassing the the *pri-miR156h* sequence was amplified introducing *Xho*I and *BamH*I restriction sites and subcloned in *ML595. MIM156h* was generated using specific primer sequences analogous to *MIM156* (Franco-Zorrilla JM, et al. (2007) Target mimicry provides a new mechanism for regulation of microRNA activity. Nature Genet 39(8):1033-1037). *HSP21::MIR156h* and *HSP21::MIM156h* were generated by replacing the *HSP21* coding sequence in a 2.8 kb genomic fragment with the *MIR156h* and *MIM156h* fragments described above. All experiments were performed with multiple homozygous lines carrying a single insertion (determined by segregation).

### RNA preparation, microarray analysis and qRT-PCR

RNA was extracted from seedlings using a Trizol-based protocol (Pant BD, et al. (2009) Identification of nutrient-responsive Arabidopsis and rapeseed microRNAs by comprehensive real-time polymerase chain reaction profiling and small RNA sequencing. Plant Physiol 150(3):1541-1555) or a hot-phenol RNA extraction protocol (Bäurle I, Smith L, Baulcombe DC, & Dean C (2007) Widespread role for the flowering-time regulators FCA and FPA in RNA-mediated chromatin silencing. Science 318(5847): 109-112). For microarray analysis, RNA from three biological replicates per genotype and treatment (4h after ACC, 52 h after ACC, 4 h NTC) was purified over an RNeasy Plant RNA extraction column (Qiagen), and processed for hybridization of Affymetrix ATH1 Genechips (NASC microarray facility). For quantitative RT-PCR, total RNA was treated with TURBO DNA-free (Ambion) and 2 µg reverse transcribed with SuperScript III (Invitrogen) according to the manufacturers' instructions. 0.1 µl cDNA was used per 10 µl QPCR reaction with GoTaq qPCR Master Mix (Promega) and an Applied Biosystems AB7300 or Roche LightCycler 480 instrument. Expression was normalized to *TUBULIN6* using the comparative CT method. Primer sequences are listed in Table 1 and in the sequence listing.

### pri-miRNA profiling, miRNA target prediction and mature miRNA detection

The pri-miRNA profiling was performed as described (Pant BD, et al. (2009) Identification of nutrient-responsive Arabidopsis and rapeseed microRNAs by comprehensive real-time polymerase chain reaction profiling and small RNA sequencing. Plant Physiol 150(3):1541-1555). The miRNA target prediction was performed using online search tools with the default settings (Ossowski S, Schwab R, & Weigel D (2008) Gene silencing in plants using artificial microRNAs and other small RNAs. Plant J 53(4):674-690; Dai X & Zhao PX (2011) psRNATarget: a plant small RNA target analysis server. Nuc Acids Res 39 (Web Server issue):W155-159; Moxon S, et al. (2008) A toolkit for analysing large-scale plant small RNA datasets. Bioinformatics 24(19):2252-2253; Alves L, Jr., Niemeier S, Hauenschild A, Rehmsmeier M, & Merkle T (2009) Comprehensive prediction of novel microRNA targets in Arabidopsis thaliana. Nuc Acids Res 37(12): 4010-4021) and published degradome data (39). For detection of mat-miRNA, a modified stem-loop qRT-PCR protocol was used with SYBR Green as a dye (60, 61).

### MUG assay

To quantify GUS protein level, 4-Methylumbelliferyl b-D-glucuronidase (MUG) activity was determined with a fluorescence spectrophotometer according to standard procedures (Jefferson RA, Kavanagh TA, & Bevan MW (1987) GUS fusions: beta-glucuronidase as a sensitive and versatile gene fusion marker in higher plants. EMBO J 6(13):3901-3907).

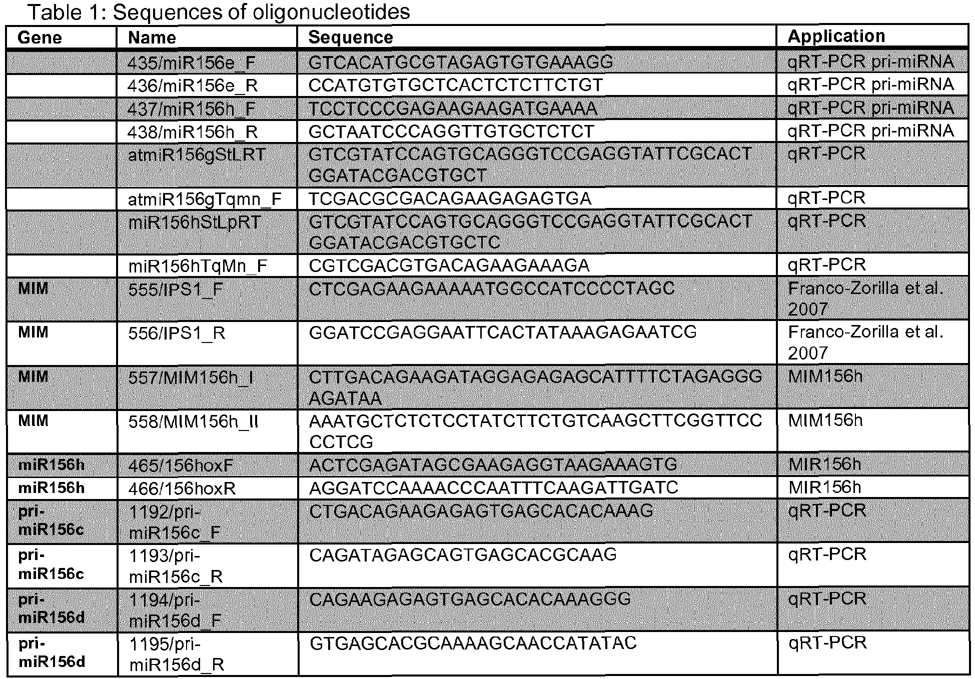

### Figures

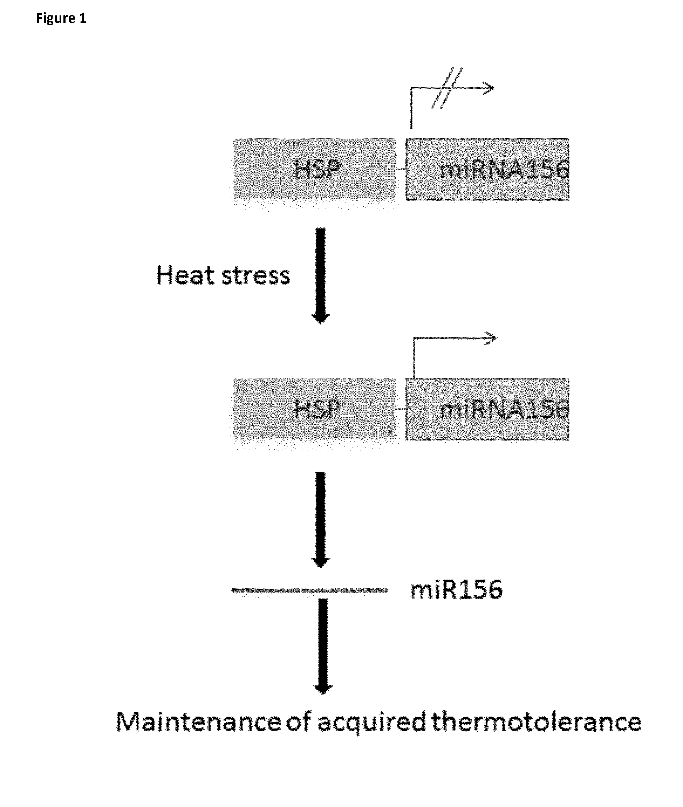

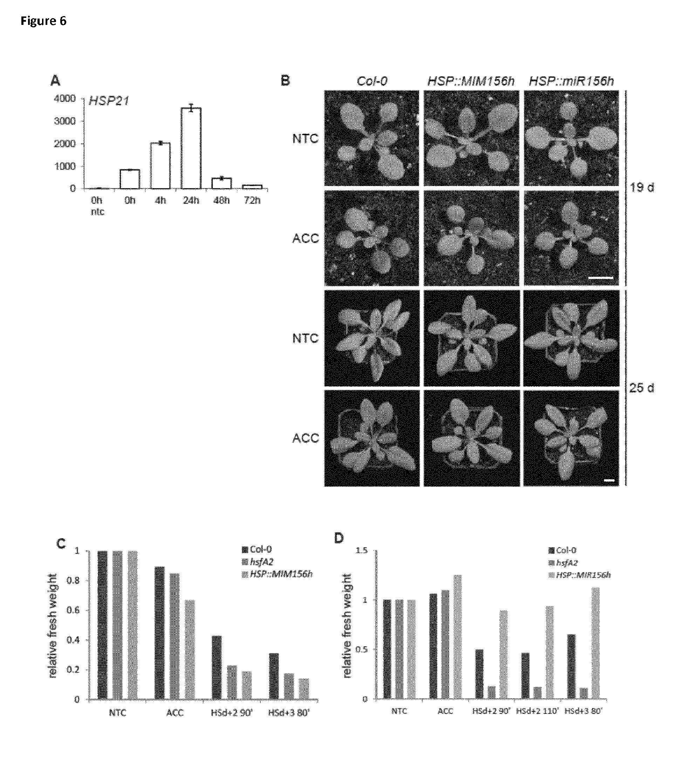

**Figure 1****: Schematic overview of the method for providing a heat-stress resistant plant.**
   Figure 1 depicts the principle of the invention. A member of the miRNA156 family of miRNAs is positioned such that it is under the control of a heat-stress inducible promotor. After introduction into a plant cell, the member of the miRNA156 family of miRNAs is expressed upon heat stress (miRNA156 is shown here as an example), rendering the plant heat-resistant.

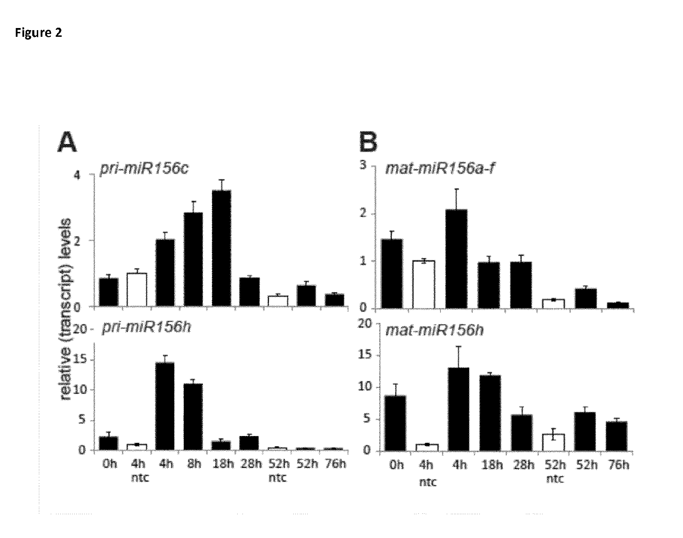

**Figure 2****: Transcript levels of HS-responsive *pri-miR156* and corresponding mature miRNA levels during maintenance of acquired thermotolerance.**
   (A) Quantitative RT-PCR for *pri-miR156c* and *pri-miR156h.*
   (B) Quantitative RT-PCR for mature *miR156a-f* and *miR156h.*
      Expression values were normalized to *TUB6* and the respective 4 h ntc. Error bars are SEM of three replicates. One representative of at least three independent experiments is shown.

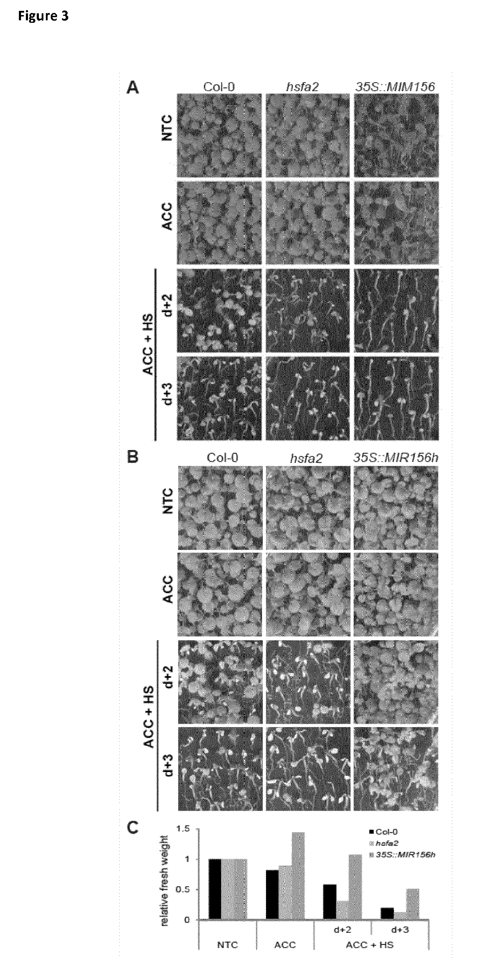

**Figure 3****: *MiR156* regulates the maintenance of acquired thermotolerance and development.**
   (A) Knockdown of *miR156a-f* in *35S::MIM156* impairs HS memory, assayed by applying a tester HS (HS) two (+2) and three days (+3) after a priming HS (ACC). Pictures were taken 14 d after ACC. All plants of one treatment were grown on the same plate.
   (B) Overexpression of *miR156h* enhances the HS memory, assayed by applying a tester HS (HS) two (+2) and three days (+3) after a short ACC. Pictures were taken 14 d after ACC. All plants of one treatment were grown on the same plate.
   (C) Quantification of (B); average seedling fresh weight was determined from 27±1 seedlings per genotype and treatment.
      The experiments were repeated at least 5 times with at least two independent transgenic lines with similar results.

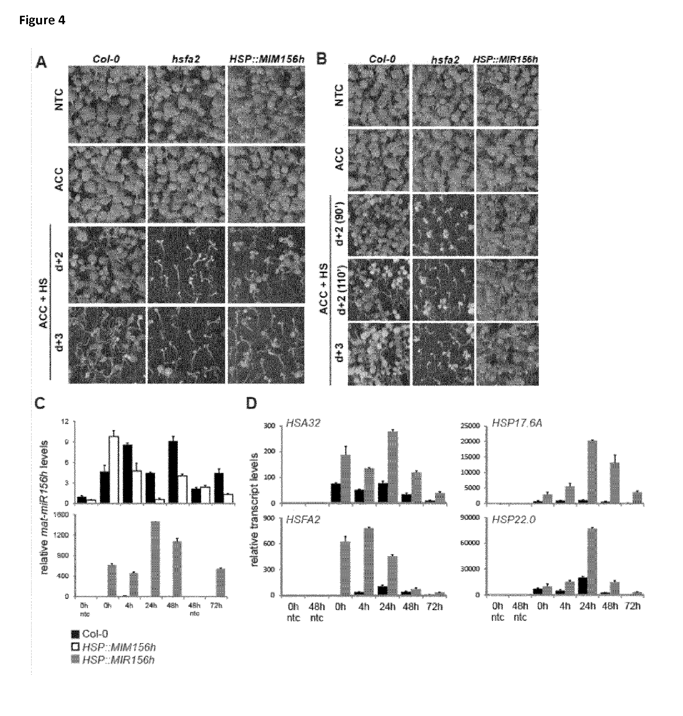

**Figure 4****: *MiR156* is required after HS to regulate the maintenance of acquired thermotolerance.**
   (A) Heat-inducible knock-down of *miR156h* (*HSP21::MIM156h*) impairs the HS memory, assayed by applying a tester HS (HS) two (+2) and three days (+3) after a priming HS (ACC). Pictures were taken 14 d after ACC. All plants of one treatment were grown on the same plate. See Fig. 6C for quantification.
   (B) Heat-inducible overexpression of *MIR156h* (*HSP21::MIR156h*) enhances the HS memory, assayed by applying a tester HS (HS) two (+2, 90, 110 min) and three days (+3) after a short ACC. Pictures were taken 14 d after ACC. All plants of one treatment were grown on the same plate. See Fig. 6D for quantification.
   (C) Mat-miRNA levels of *miR156h* in *HSP::MIR156h* and *HSP::MIM156h* after ACC determined by qRT-PCR. Error bars are SEM of three replicates. Three independent experiments were performed with one representative shown.
   (D) Expression of selected HS-memory related genes determined by qRT-PCR is increased after a priming HS in *HSP::MIR156h.* Expression values were normalized to *TUB6* and 48 h ntc. Error bars are SEM of three replicates. Three independent experiments were performed with one representative shown.

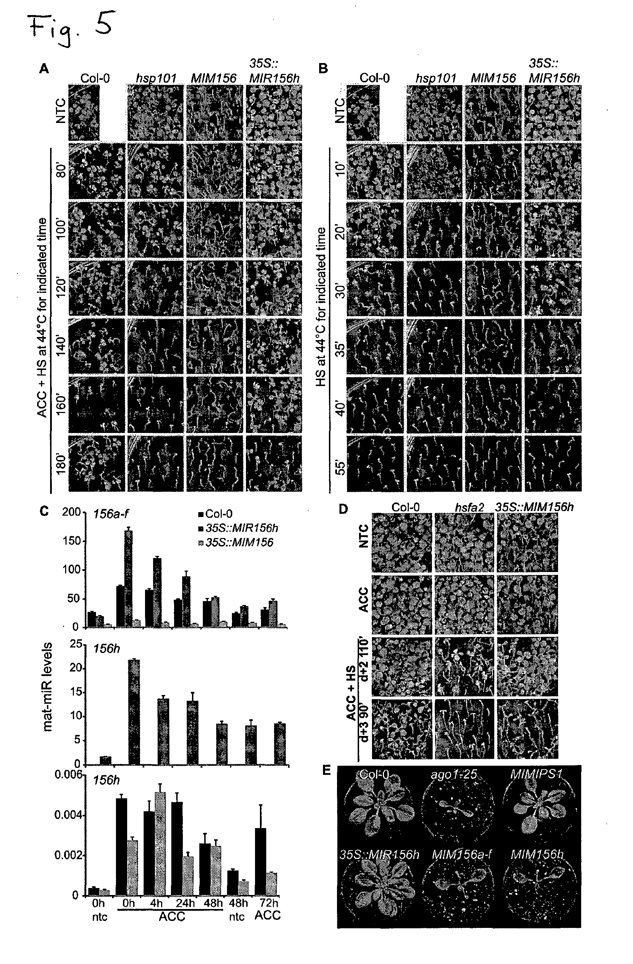

**Figure 5****: Further characterization of *35S::MIM156* and *35S::miR156h.***
   (A) Acquisition of thermotolerance in *35S::MIM156a-f* and *35S::MIR156h* was assayed by extending the second HS of the priming HS treatment for the indicated times. Pictures were taken 8 d after the HS.
   (B) Basal thermotolerance was assayed by incubating 4 day old seedlings for the indicated times at 44°C. Pictures were taken 8 d after the HS.
   (C) Mat-miRNA levels of *miR156a-f* and *miR156h* in Col (black), *35S::MIR156h* (blue) and *35S::MIM156* (green) determined by qRT-PCR. Error bars are SEM of three replicates. The experiment was repeated at least twice independently and one representative is shown.
   *(D) 35S::MIM156h* plants were assayed for the maintenance of acquired thermotolerance by applying a tester HS (HS) two (+2, 90 min) and three days (+3, 90 min) after a priming HS (ACC). Pictures were taken 14d after ACC.
   (E) Effects of *35S::MIM156a-f, 35S::MIM156h* and *35S::miR156h* on rosette phenotypes of 22 d old plants grown in long days. Size bar is 1 cm. *MIMIPS1* [Fornara F, de Montaigu A, & Coupland G (2010) SnapShot: Control of flowering in Arabidopsis. Cell 141(3):550, 550 e551-552] was included as a control.
      All plants of one treatment were grown on the same plate. The result is representative for several experiments with homozygous lines derived from multiple independent transformants.
**Figure 6****: Further characterization of heat-inducible miR156-manipulating lines.**
   (A) Transcript levels of *HSP21* after a priming HS as determined by qRT-PCR normalized to *TUB6* and 0h ntc.
   (B) 19 and 25 day-old rosette plants of the indicated genotypes treated with a priming HS (ACC) on day 4 or mock-treated (NTC). Size bars are 1 cm.
   (C) Quantification of Figure 4A; average seedling fresh weight was determined from 32±3 seedlings per genotype and treatment.
   (D) Quantification of Figure 6B; average seedling fresh weight was determined from 24±3 seedlings per genotype and treatment.

## Claims

1. A polynucleotide, comprising
- A first sequence portion for driving transcription of a second sequence portion, and
- A second sequence portion encoding a miRNA of the plant miRNA156 family,
wherein the first sequence portion is
- heat-stress inducible and
- operably linked to the second sequence portion.

2. The polynucleotide of claim 1, wherein the first sequence portion is a promotor selected from the group consisting of: HSP21, HSA32, HSP17, HSP18, HSP22, and HSP70.

3. The polynucleotide of claim 1 or 2, wherein the miRNA of the plant miRNA156 family comprises a sequence selected from the group consisting of: miRNA156a-f, miRNA156g, and miRNA156h, and miR157a-d.

4. The polynucleotide of claims 1 to 4, wherein the polynucleotide is a DNA, an RNA, or a chemically modified DNA or RNA.

5. The polynucleotide of claims 1 to 4, wherein the polynucleotide stems from or is derived from a plant selected from a monocotyledonous plant or a dicotyledonous plant.

6. The polynucleotide of claim 5, wherein the a monocotyledonous plant that is selected from the group consisting of rice, wheat, barley, oats, rye, sorghum, maize, sugarcane, pineapple, onion, bananas, coconut, lilies, turfgrasses, and millet; and
wherein the dicotyledonous plant that is selected from the group consisting of tomato, cucumber, squash, peas, alfalfa, melon, chickpea, chicory, clover, kale, lentil, soybean, beans, tobacco, potato, sweet potato, yams, cassava, radish, broccoli, spinach, cabbage, rape, apple trees, citrus (including oranges, mandarins, grapefruit, lemons, limes and the like), grape, cotton, sunflower, strawberry, lettuce, and hop.

7. A vector comprising a polynucleotide of claims 1 to 6, in particular wherein the vector is a plasmid, an artificial chromosome or a cosmid.

8. A heat-resistant plant cell comprising a polynucleotide of claims 1 to 6.

9. The heat-resistant plant cell of claim 8, wherein the polynucleotide is located chromosomally or exrachomosomally.

10. A heat-resistant plant, comprising a polynucleotide of claims 1 to 6.

11. The heat-resistant plant of claim 10, wherein the polynucleotide is located chromosomally or exrachomosomally.

12. The plant cell or plant of claims 8 or 11,
wherein the plant is a monocotyledonous plant or plant tissue or cell that is selected from the group consisting of: rice, wheat, barley, oats, rye, sorghum, maize, sugarcane, pineapple, onion, bananas, coconut, lilies, turfgrasses, and millet; or
wherein the plant is a dicotyledonous plant or plant tissue or cell that is selected from the group consisting of: tomato, cucumber, squash, peas, alfalfa, melon, chickpea, chicory, clover, kale, lentil, soybean, beans, tobacco, potato, sweet potato, yams, cassava, radish, broccoli, spinach, cabbage, rape, apple trees, citrus (including oranges, mandarins, grapefruit, lemons, limes and the like), grape, cotton, sunflower, strawberry, lettuce, and hop.

13. A method for generating a heat-resistant plant, in particular a plant of claim 8 or 9, comprising the step of generating a polynucleotide of claims 1 to 7 in a plant cell or of introducing a polynucleotide of claims 1 to 7 into a plant cell.

14. The method of claim 13, further comprising the step of subjecting the plant to a heat stress.

15. A method claim 13 to 14, further comprising the step of selecting a plant in which the polynucleotide of claims 1 to 5 is integrated into the genome of the plant.


SEQUENCE LISTING

<110> UniversitÃ¤t Potsdam

<120> METHOD AND MATERIALS FOR MAKING PLANTS HEAT-RESISTANT

<130> BRA008EP

<150> EP13192897.0
<151> 2013-11-14

<160> 12

<170> BiSSAP 1.2

<210> 1
<211> 20
<212> DNA
<213> Arabidopsis thaliana

<220>
<221> source
<222> 1..20
<223> /organism="Arabidopsis thaliana"
      /mol_type="unassigned DNA"

<400> 1
tgacagaaga gagtgagcac                                                   20


<210> 2
<211> 20
<212> DNA
<213> Arabidopsis thaliana

<220>
<221> source
<222> 1..20
<223> /organism="Arabidopsis thaliana"
      /mol_type="unassigned DNA"

<400> 2
cgacagaaga gagtgagcac                                                   20


<210> 3
<211> 20
<212> DNA
<213> Arabidopsis thaliana

<220>
<221> source
<222> 1..20
<223> /organism="Arabidopsis thaliana"
      /mol_type="unassigned DNA"

<400> 3
tgacagaaga aagagagcac                                                   20


<210> 4
<211> 21
<212> DNA

<213> Arabidopsis thaliana

<220>
<221> source
<222> 1..21
<223> /organism="Arabidopsis thaliana"
      /mol_type="unassigned DNA"

<400> 4
ttgacagaag atagagagca c                                                21


<210> 5
<211> 21
<212> DNA
<213> Arabidopsis thaliana

<220>
<221> source
<222> 1..21
<223> /organism="Arabidopsis thaliana"
      /mol_type="unassigned DNA"

<400> 5
ttgacagaag atagagagca c                                                21


<210> 6
<211> 21
<212> DNA
<213> Arabidopsis thaliana

<220>
<221> source
<222> 1..21
<223> /organism="Arabidopsis thaliana"
      /mol_type="unassigned DNA"

<400> 6
ttgacagaag atagagagca c                                                21


<210> 7
<211> 20
<212> DNA
<213> Arabidopsis thaliana

<220>
<221> source
<222> 1..20
<223> /organism="Arabidopsis thaliana"
      /mol_type="unassigned DNA"

<400> 7
tgacagaaga tagagagcac                                                  20


<210> 8
<211> 1299
<212> DNA
<213> Arabidopsis thaliana

<220>

<221> source
<222> 1..1299
<223> /organism="Arabidopsis thaliana"
      /mol_type="unassigned DNA"

<400> 8
tcaagaaatt agggttccca gatgaaaaag aggatatgtt agcagaagac ggaaacaaat      60

ttggttgttc ttgtggacga cgacagagct tttgccttgg tgggggtttc tcgaatactt     120

ggagatcgag gaaaggagaa gctgaaatta ccgaaatagc cttagctgca ccgtcgtaag     180

tcgtgtaact agattgtgtc aagaagggcc tttaataggc cttcaattca gaatgttcta     240

aaaccgaatt aaagtactgt tcggcgtcca ctagattttg taggtactat tgcatcatca     300

tcatcttata tcagttcaat gtgtgtcttg tttttctttt tttagtctat tacatcatct     360

gatcttattc tagcagcctt ttgaaaatat agagaaaaat cattcaatgt ttctaaaacc     420

aaatctatac acaaaacata aagtcttttg gatcattgaa catttgatcg gattgcggat     480

taatagatta gagcttaaat tttgtataat tttataccaa tcaaaattaa tatagagtca     540

tgtcatgcta tttgtataat ttatgtacaa caattattat ataaaacgac ttgaacaatt     600

ctgtgataaa gaaaaaaaaa aaaaaaacga cttgaacaat tcaacatgct atagatagtt     660

tatacgtaag ctaataagcc tgtttgatac tgtatatgac atacgtgtag ctgatatgat     720

caaactagac aaaatttctt aatttctccc gtagatatat atttttagtt tacttgggca     780

ttccatttct catatgtttc tgcaagttag cacacataat ggtgacttgg tgagttgctt     840

tttttctctc tctttttcaa tatgatatgc tttcttcgat ttttttattt tttttaaaca     900

atatatcatc taatcatggt tttttcgtaa tcgtggtttt tctaattgta gaaatctagt     960

cttttttttt cgtttatttt gtttttatta gttcatcaca aataaacact gattggcaag    1020

aaggaaaaaa agaagatggt tcgggagaag atgagaaaac acgagaaaac gcacaaaggt    1080

taacaaatag taaaatagat agatagaaaa caaaacgagt cgagaaactt cgtatcaaat    1140

cttcaacctt caagacctct ccttttgtca cttagagcct tctcatcctc acctacatat    1200

atacactcac ttctacaatt gtagtagata ctcaacaaac tcagaactag aattttctaa     1260

tcttcactca gcgatatttt tggctcatac tcgaaacaa                           1299

<210> 9
<211> 659
<212> DNA
<213> Hordeum vulgare

<220>
<221> source
<222> 1..659
<223> /organism="Hordeum vulgare"
      /mol_type="unassigned DNA"

<400> 9

gtagagaatt ttaagatgtc catgaccacc cgaccacccg ctagctgcgt cattgtgtag 60

tactctttct gtttttaaaa taagtgtctg aactttatac taactttaat ataaaattgt 120

actaaacttg agacatttat ttagacggag ggagtagtat ttatttacgt ttaactattg 180

tacttgctcg ctgtaaactt taatgacaga taacctctac tgttaatcat gctattctgg 240

aaacggcccc acaacccatt cttgaatcta gactagtcgc ccccttgctg gaattataca 300

attatttata gaaaaatctc acaaaaaaat tattaacaaa aaaaaaaggg agaaggcgcc 360

ctaacggcag gttcgtgagt cccctggaag acgatagacc cttcgagggt tgggaacagg 420

aaagaacgaa tggctcacag cctacctgga aagcgaaaga gaaacaaaca ctcgtggatt 480

ggacagtcct cgagattctt caagatcagt acctacgatg ctataagtag tggctcacct 540

tccatcgccc tcccaactca agccagccag cgtcaaacca aagcgaacaa catcctacct 600

acaatccgat acagattaat ttcaacgcga gctacaaacc aaagcaagca acaccgaca 659

<210> 10
<211> 135
<212> RNA
<213> Hordeum vulgare

<220>
<221> source
<222> 1..135
<223> /organism="Hordeum vulgare"
/mol_type="unassigned RNA"

<400> 10
ugacagaaga gagugagcac acagcggcag gcuacgucug auaagagcau gguuaauagu 60

auagccaacu gcuggcuaua cuaugagcac gugcuuagac gcagcugcug uugcgugcuc 120

acccucucuc ugucu 135

<210> 11
<211> 21
<212> RNA
<213> Hordeum vulgare

<220>
<221> source
<222> 1..21
<223> /organism="Hordeum vulgare"
/mol_type="unassigned RNA"

<400> 11
ugacagaaga gagugagcac a 21

<210> 12
<211> 21
<212> DNA
<213> Brassica napus

<220>

<221> source
<222> 1..21
<223> /organism="Brassica napus"
      /mol_type="unassigned DNA"

<400> 12
ugacagaaga gagugagcac a                                                21